# EUROPÄISCHE PATENTSCHRIFT

(11) **EP 0 583 589 B1**
(45) Veröffentlichungstag und Bekanntmachung des Hinweises auf die Patenterteilung: **12.06.1996**
(21) Anmeldenummer: 93110678.5
(22) Anmeldetag: 05.07.1993
(51) Int. Cl.: C07C 51/58, C07C 63/70

(54) **Verfahren zur Herstellung von aromatischen o-Chlormethylcarbonsäurechloriden**
Process for the preparation of aromatic o-chloromethyl carboxylic acid chlorides
Procédé pour la préparation de chlorures d'acides o-chlorométhyl-carboxyliques aromatiques

(30) Priorität: 16.07.1992 DE 4223382
(43) Veröffentlichungstag der Anmeldung: 23.02.1994
(73) Patentinhaber: BASF Aktiengesellschaft, D-67063 Ludwigshafen (DE)
(72) Erfinder: Wettling, Thomas, Dr., D-6703 Limburgerhof (DE); Henkelmann, Jochem, Dr., D-6700 Ludwigshafen (DE); Troetsch-Schaller, Irene, Dr., D-6710 Frankenthal (DE)

(56) Entgegenhaltungen:
- EP-A- 0 253 214
- DE-C- 804 567
- US-A- 2 053 269
- US-A- 2 778 852
- DATABASE WPI Week 6800, Derwent Publications Ltd., London, GB; AN 68-79620P & JP-B-42 004 329 (SEISAN KAIHATSU KK)
- J.C.S. CHEM. COMM. Nr. 13, 1973, Seite 425 D.J. BURTON ET AL
- CURRENT SCIENCE Bd. 55, Nr. 13, 1986, Seiten 614 - 615 V. GOPALAKRISHNAN ET AL

## Beschreibung

Die vorliegende Erfindung betrifft ein Verfahren zur Herstellung von aromatischen o-Chlormethylcarbonsäurechloriden durch Umsetzung von benzokondensierten Lactonen mit Phosgen in Gegenwart eines Katalysators wie z.B. einer organischen Stickstoffverbindung.

Die Herstellung von nicht aromatischen Chlorcarbonsäurechloriden aus aliphatischen oder cycloaliphatischen Lactonen und Phosgen bei erhöhten Temperaturen ist aus der US-A-2 778 852 mit Pyridin als Katalysator, aus der DE-A-36 24 258 mit quartären Ammoniumsalzen und aus der DE-A-39 27 146 mit Phosphinoxiden als Katalysator bekannt.

Die Herstellung aromatischer o-Chlormethylcarbonsäurechloride mit Hilfe von Phosgen ist bislang nicht bekannt.

Der Erfindung lag daher die Aufgabe zugrunde, diese Reaktion auf benzokondensierte Lactone zu übertragen.

Demgemäß wurde ein neues und verbessertes Verfahren zur Herstellung von aromatischen o-Chlormethylcarbonsäurechloriden der allgemeinen Formel I 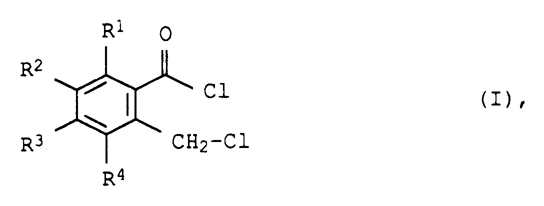
in der
- R¹,R²,R³,R⁴: Wasserstoff, C₁- bis C₂₀-Alkyl, Aryl, oder Halogen oder R¹ und R², R¹ und R³, R¹ und R⁴, R² und R³, R² und R⁴ oder R³ und R⁴ gemeinsam eine C₁-bis C₆-Alkylenkette oder But-1,3-dienyl
bedeuten, gefunden, welches dadurch gekennzeichnet ist, daß man die Umsetzung von benzokondensierten Lactonen der allgemeinen Formel II 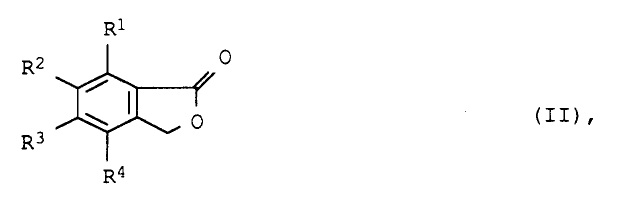
in der R¹, R², R³ und R⁴ die oben genannten Bedeutungen haben, mit Phosgen bei Temperaturen von 100 bis 240°C in Gegenwart eines Katalysators durchführt.

Das erfindungsgemäße Verfahren läßt sich wie folgt durchführen:
Das benzokondensierte Lacton II und der Katalysator können gegebenenfalls in einem inerten Lösungsmittel vorgelegt werden und bei Temperaturen von 100 bis 240°C, bevorzugt 130 bis 200°C, besonders bevorzugt 160 bis 190°C und Drücken von 0,01 bis 50 bar, bevorzugt 0,1 bis 5 bar, besonders bevorzugt Normaldruck (Atmosphärendruck) mit Phosgen zur Reaktion gebracht werden. Das Verfahren kann diskontinuierlich oder bevorzugt kontinuierlich durchgeführt werden.

Als Katalysator eignen sich organische Stickstoffverbindungen z.B. Stickstoffheterocyclen wie Pyridin, Imidazol, Chinolin, Morpholin, Pyrimidin, Pyrazin, Indol und Pyrazol, und deren alkylsubstituierte Derivate wie 1-Methylimidazol, 1-Decylimidazol, N-Methylmorpholin und 1-Methylpyrazol, bevorzugt alkylsubstituierte Pyridinderivate wie 3-Methylpyridin, 2-Methylpyridin, 4-Methylpyridin, 2,3-Dimethylpyridin, 2,4-Dimethylpyridin und 3,4-Dimethylpyridin, acyclische Stickstoffverbindungen z.B. alkylsubstituierte Amine wie Triethylamin, Tri-n-propylamin, Triisopropylamin, Tri-n-butylamin und Triisobutylamin, bevorzugt Tri-n-butylamin und Triisobutylamin, Ammoniumsalze wie Tetramethylammoniumchlorid, Tetrabutylammoniumchlorid und Benzyltrimethylammoniumchlorid, bevorzugt Tetrabutylammoniumchlorid, Harnstoffe wie N,N,N',N'-Tetramethylharnstoff und N,N,N',N'-Tetra-n-butylharnstoff, bevorzugt Tetra-n-butylharnstoff und Guanidine wie N,N,N',N'-Tetraphenylguanidin und N,N,N',N'-Tetramethyl-N-phenylguanidin, bevorzugt alkylsubstituierte Guanidinderivate, insbesondere N,N,N',N'-Tetramethylguanidin, Formamide wie Dimethylformamid, Diethylformamid, Di-n-phenylformamid, Di-iso-propylformamid, Di-n-butylformamid, Di-sec.-butylformamid und Di-iso-butylformamid, bevorzugt Dimethylformamid und Di-sec.-butylformamid.

Das Molverhältnis von Katalysator zum benzokondensierten Lacton II beträgt 0,005 : 1 bis 0,1 : 1, bevorzugt 0,01 : 1 bis 0,05 : 1.

Das Molverhältnis von Phosgen zum benzokondensierten Lacton II beträgt 0,8 : 1 bis 10 : 1, bevorzugt 1 : 1 bis 3 : 1, besonders bevorzugt 1 : 1 bis 1,5 :1, wobei man im Falle eines vorteilhaften Phosgenüberschußes diesen vorteilhaft durch Kühlung der Abgase auf unter (-40°C), vorzugsweise unter (-70°C) auskondensiert und in das Reaktionsgefäß zurückleitet.

Als inerte Lösungsmittel eignen sich solche Lösungsmittel, die unter den Reaktionsbedingungen nicht mit den Ausgangsstoffen und den Reaktionsprodukten reagieren, das sind z.B. hochsiedende Kohlenwasserstoffe wie Cumol, Paraffinöl und Naphthalin sowie aromatische Chlorverbindungen wie Dichlorbenzol und Trichlorbenzol.

Im allgemeinen ist ein Lösungsmittel jedoch nicht erforderlich.

Nach einer besonders vorteilhaften Ausführungsform leitet man gleichzeitig mit dem Phosgen bis zu 100 Mol-%, bezogen auf das Lacton, an Chlorwasserstoff oder an solchen Verbindungen in das Reaktionsgefäß ein, die mit Phosgen Chlorwasserstoff bilden, wie z.B. Wasser. Vorzugsweise gibt man 5 bis 100, insbesondere 20 bis 40 Mol-% Chlorwasserstoff oder Chlorwasserstoff bildende Stoffe, bezogen auf das Lacton zu.

Überraschenderweise hat sich gezeigt, daß durch diese Zusätze die Umsetzung des benzokondensierten Lactons zu den aromatischen o-Chlormethylcarbonsäurechloriden I stark beschleunigt wird. Schon kleine Mengen dieser Zusätze zeigen einen deutlichen Effekt. Größere Mengen an Chlorwasserstoff sind aus wirtschaftlichen Gründen und wegen des Strippeffektes auf das im Reaktionsgemisch gelöste Phosgen nicht zu empfehlen.

Die Substituenten R¹, R², R³ und R⁴ in den Formeln I und II haben die folgenden Bedeutungen:
R¹,R²,R³,R⁴
- Wasserstoff,
- C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- Halogen wie Fluor, Chlor, Brom und Jod, bevorzugt Fluor, Chlor und Brom, besonders bevorzugt Fluor und Chlor,
R¹ und R², R¹ und R³, R¹ und R⁴,
R² und R³, R² und R⁴ oder R³ und R⁴
- gemeinsam eine
- C₁- bis C₆-Alkylenkette wie -(CH₂)-, -(CH₂)₂-, -(CH₂)₃-, -(CH₂)₄-, -(CH₂)₅- und -(CH₂)₆-, bevorzugt -(CH₂)₄.
- But-1,3-dienyl.

### Beispiele

### Beispiel 1 bis 6

In einem Rührreaktor mit Trockeneisrückflußkühlung und Zuführmöglichkeit von Chlorwasserstoff und Phosgen werden x g Phthalid und 5 Mol-% organische Stickstoffverbindung vorgelegt und die Innentemperatur durch ein Heizmedium auf 170 bis 180°C gebracht. Bei dieser Temperatur werden im Laufe von t Stunden y g Phosgen und ca. 10 l/h gasförmiger Chlorwasserstoff eingeleitet. Aus dem Abgas wird das Phosgen durch Kühlung auf (-70°C) auskondensiert und in die Reaktionszone zurückgeführt. Nach beendeter Reaktion wird das Reaktionsgemisch zur Vervollständigung der Reaktion noch 1 Stunde bei dieser Temperatur gehalten. Danach bläst man mit Stickstoff überschüssiges Phosgen aus und destilliert das o-Chlormethylbenzoesäurechlorid über eine Kolonne bei 100°C/1 mbar ab.

Die genauen Angaben zu den einzelnen Beispielen können nachfolgender Tabelle entnommen werden.

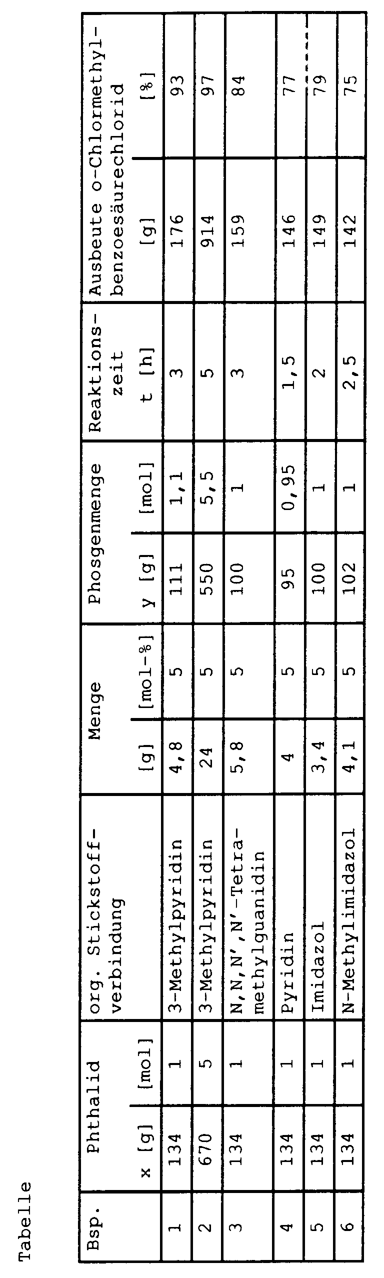

## Patentansprüche

1. Verfahren zur Herstellung von aromatischen o-Chlormethylcarbonsäurechloriden der allgemeinen Formel I 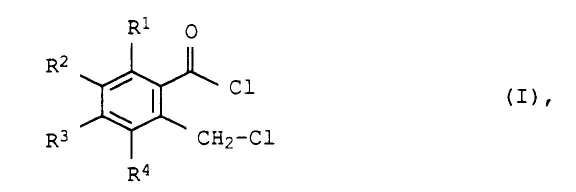 in der
R¹,R²,R³,R⁴ Wasserstoff, C₁- bis C₂₀-Alkyl, Aryl, oder Halogen oder R¹ und R², R¹ und R³, R¹ und R⁴, R² und R³, R² und R⁴ oder R³ und R⁴ gemeinsam eine C₁- bis C₆-Alkylenkette oder But-1,3-dienyl bedeuten, dadurch gekennzeichnet, daß man die Umsetzung von benzokondensierten Lactonen der allgemeinen Formel II 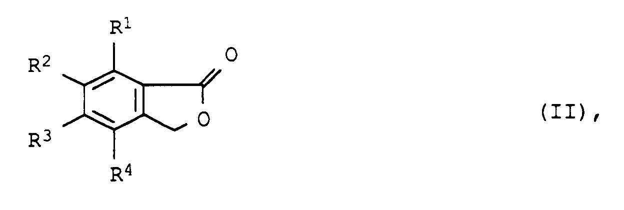
in der R¹, R², R³ und R⁴ die oben genannten Bedeutungen haben, mit Phosgen bei Temperaturen von 100 bis 240°C in Gegenwart eines Katalysators durchführt.

2. Verfahren nach Anspruch 1, dadurch gekennzeichnet, daß man als benzokondensiertes Lacton Phthalid verwendet.

3. Verfahren nach Anspruch 1, dadurch gekennzeichnet, daß man als Katalysator eine organische Stickstoffverbindung verwendet.

4. Verfahren nach Anspruch 1, dadurch gekennzeichnet, daß man als organische Stickstoffverbindung 3-Methylpyridin oder N,N,N',N'-Tetramethylguanidin verwendet.

5. Verfahren nach Anspruch 1, dadurch gekennzeichnet, daß man die Umsetzung bei 130 bis 200°C durchführt.

6. Verfahren nach Anspruch 1, dadurch gekennzeichnet, daß man den zugesetzten Katalysator in Mengen von 0,5 bis 10 Mol-%, bezogen auf das eingesetzte benzokondensierte Lacton, verwendet.

7. Verfahren nach Anspruch 1, dadurch gekennzeichnet, daß man 5 bis 100 Mol-%, bezogen auf das Lacton, an Chlorwasserstoff oder eine Chlorwasserstoff bildende Verbindung einsetzt.

8. Verfahren nach Anspruch 1, dadurch gekennzeichnet, daß man den zugesetzten Katalysator, der im Produktdestillationsrückstand verbleibt, wiederverwendet.

## Claims

1. A process for the preparation of aromatic o-chloromethylcarbonyl chlorides of the formula I 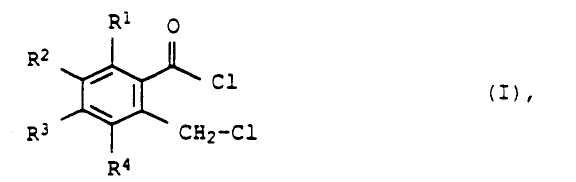 where
R¹, R², R³ and R⁴ are hydrogen, C₁- to C₂₀-alkyl, aryl or halogen, or R¹ and R², R¹ and R³, R¹ and R⁴, R² and R³, R² and R⁴ or R³ and R⁴ together are a C₁- to C₆-alkylene chain or but-1,3-dienyl,
which comprises carrying out the reaction of benzo-fuzed lactones of the formula II 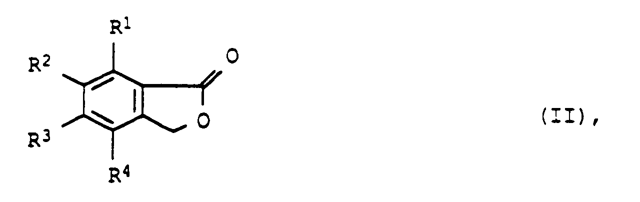 where R¹, R², R³ and R⁴ are as defined above, with phosgene at from 100 to 240°C in the presence of a catalyst.

2. A process as claimed in claim 1, wherein the benzo-fuzed lactone used is phthalide.

3. A process as claimed in claim 1, wherein the catalyst used is an organonitrogen compound.

4. A process as claimed in claim 1, wherein the organonitrogen compound used is 3-methylpyridine or N,N,N',N'-tetramethylguanidine.

5. A process as claimed in claim 1, wherein the reaction is carried out at from 130 to 200°C.

6. A process as claimed in claim 1, wherein the added catalyst is used in an amount of from 0.5 to 10 mol%, based on the benzo-fuzed lactone employed.

7. A process as claimed in claim 1, wherein from 5 to 100 mol%, based on the lactone, of hydrogen chloride or a compound which forms hydrogen chloride are employed.

8. A process as claimed in claim 1, wherein the added catalyst remaining in the product distillation residue is re-used.

## Revendications

1. Procédé de préparation de chlorures d'acides o-chlorométhylcarboxyliques de la formule générale I 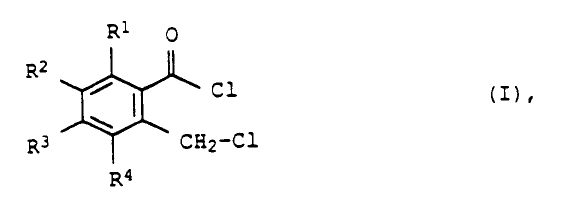 dans laquelle
R¹, R², R³, R⁴ représentent chacun un atome d'hydrogène, un radical alkyle en C₁-C₂₀, aryle ou un atome d'halogène, ou bien R¹ et R², R¹ et R³, R¹ et R⁴, R² et R³, R² et R⁴, ou R³ et R⁴ représentent ensemble un chaîne alkylène en C₁ à C₆ ou le radical but-1,3-diényle, caractérisé en ce que l'on entreprend la réaction de lactones benzocondensées de la formule générale II 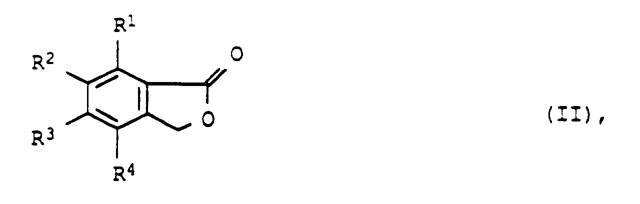
dans laquelle R¹, R², R³ et R⁴ ont les significations qui leur ont été attribuées ci-dessus, avec le phosgène à des températures de 100 à 240°C et en présence d'un catalyseur.

2. Procédé suivant la revendication 1, caractérisé en ce que l'on utilise le phtalide à titre de lactone benzocondensée.

3. Procédé suivant la revendication 1, caractérisé en ce que l'on utilise un composé azoté organique à titre de catalyseur.

4. Procédé suivant la revendication 1, caractérisé en ce que l'on utilise la 3-méthylpyridine ou la N,N,N',N'-tétraméthylguanidine à titre de composé azoté organique.

5. Procédé suivant la revendication 1, caractérisé en ce que l'on entreprend la réaction à 130-200°C.

6. Procédé suivant la revendication 1, caractérisé en ce que l'on utilise le catalyseur ajouté en proportions de 0,5 à 10% molaires, par rapport à la lactone benzocondensée mise en oeuvre.

7. Procédé suivant la revendication 1, caractérisé en ce que l'on utilise de 5 à 100% molaires, par rapport à la lactone, de chlorure d'hydrogène, ou d'un composé libérant du chlorure d'hydrogène.

8. Procédé suivant la revendication 1, caractérisé en ce que l'on réutilise le catalyseur ajouté qui subsiste dans le résidu de distillation du produit.
